Europäisches Patentamt

European Patent Office

Office européen des brevets

(11) Veröffentlichungsnummer: **0 316 593 B1**

(19)

(12) **EUROPÄISCHE PATENTSCHRIFT**

(45) Veröffentlichungstag der Patentschrift: **01.04.92**

(51) Int. Cl.⁵: **A61M 13/00**

(21) Anmeldenummer: **88117297.7**

(22) Anmeldetag: **18.10.88**

(54) **Insufflationsgerät für endoskopische Eingriffe.**

(30) Priorität: **17.11.87 DE 3739003**

(43) Veröffentlichungstag der Anmeldung:
**24.05.89 Patentblatt 89/21**

(45) Bekanntmachung des Hinweises auf die
Patenterteilung:
**01.04.92 Patentblatt 92/14**

(84) Benannte Vertragsstaaten:
**DE FR GB IT NL**

(56) Entgegenhaltungen:
**EP-A- 0 169 972**
**DE-A- 3 000 218**
**US-A- 3 897 682**
**US-A- 4 464 169**

(73) Patentinhaber: **Richard Wolf GmbH**
**Pforzheimer Strasse 32**
**W-7134 Knittlingen(DE)**

(72) Erfinder: **Baier, Manfred**
**Max-von-Laue-Strasse 7**
**W-7518 Bretten(DE)**

(74) Vertreter: **Wilcken, Thomas, Dipl.-Ing. et al**
**Musterbahn 1**
**W-2400 Lübeck(DE)**

# Beschreibung

Die Erfindung geht aus von einem Insufflationsgerät gemäß dem Oberbegriff des Patentanspruchs 1.

Ein derartiges Insufflationsgerät ist aus der EP-A-0 169 972 bekannt. Bei diesem Gerät wird das Gas aus einem Gasbehälter über einen Zwischenbehälter mittels einer Zuführungsleitung zu einem Insufflationsinstrument geführt, z.B. einer Kanüle, die z.B. in die Bauchhöhle eines Patienten eingeführt wird, um die Bauchdecke für laparoskopische Eingriffe von den inneren Organen durch Insufflation eines geeigneten Gases abzuheben, um dadurch einen freien Raum für die in den Bauchraum einzuführenden Instrumente zu schaffen. In die Zuführungsleitung sind wenigstens ein Druckminderer, wenigstens ein Absperrventil, ein Druckmeßgerät und eine Flowanzeige eingegliedert und weiterhin ist die Zuführungsleitung auf einem Streckenabschnitt in zwei parallele Strömungswege aufgeteilt. Ferner ist eine Auswerteschaltung vorgesehen, die mit dem Körperhöhlendruck des Patienten in Wirkverbindung steht. Wenn bei Anwendung dieses Gerätes der maximale Gasdruck in der Körperhöhle des Patienten erreicht ist, bewirkt die Auswerteschaltung eine Umleitung des einzuleitenden Gases durch den anderen parallelen Strömungsweg hindurch, und zwar dadurch, daß das Absperrventil des ersten Strömungsweges geschlossen und dasjenige des zweiten Strömungsweges geöffnet wird. Die Versorgung der Körperhöhle mit Druckgas erfolgt dann ständig über den zweiten Strömungsweg.

In der DE-A- 30 00 218 ist ein ähnliches Insufflationsgerät offenbart.

Werden bei den bekannten Insufflationsgeräten weitere Zusatzgeräte angewendet, so kann unter Umständen durch Fehlfunktionen, falsche Bedienung oder eine falsche Handhabung sehr schnell ein zu hoher Körperhöhlendruck auftreten, der für den Patienten eine erhebliche Gefahr darstellen kann. Ein Schutz vor derart unzulässigen Druckerhöhungen bzw. Drucküberschreitungen ist bei diesen bekannten Geräten nicht oder nur in unzureichendem Maße vorhanden. Ein weiterer Nachteil der bekannten Insufflationsgeräte besteht darin, daß der durch einen auftretenden Gasverlust resultierende Druckabfall in der Körperhöhle erst nach Erreichen eines unteren vorbestimmten Grenzwertes vom System erkannt und ausgeglichen wird. Der Gasdruck in der Bauchhöhle wird dann nicht konstant, sondern lediglich in einem unzureichenden Maße aufrechterhalten und pendelt ständig zwischen einem oberen und einem unteren Grenzwert hin und her. Weiterhin ist es bei den bekannten Geräten nicht möglich, vorhandene Fehlfunktionen durch einen Test am Gerät zu erkennen.

Die Aufgabe der Erfindung besteht darin, ein Insufflationsgerät der einleitend angeführten Art so zu verbessern, daß für den Patienten gefährliche Druckerhöhungen in der Körperhöhle vermieden werden, daß die in der Körperhöhle geschaffene Gasblase auch bei plötzlichen oder ständigen Gasverlusten mit hoher Geschwindigkeit auf konstantem Druckwert gehalten wird und daß Störungen im Druckgleichgewicht erkennbar sind und ein Zustand eingenommen wird, der eine Gefährdung des Patienten ausschließt.

Die Lösung der Aufgabe ist in dem zweiten Teil des Patentanspruchs 1 angegeben.

Mit dem erfindungsgemäßen Insufflationsgerät ist es nun vermieden, daß bei Fehlfunktionen oder falscher Bedienung des Gerätes und/oder zugeschalteter Einrichtungen unzulässige Druckerhöhungen in der Körperhöhle bzw. Bauchhöhle des Patienten auftreten. Des weiteren wird ein plötzlicher Druckabfall in der Körperhöhle vermieden, denn in einem solchen Fall wird sofort auf den ersten Strömungsweg zurückgeschaltet, um Druckgas schnell nachzufüllen, und dann wieder auf den zweiten Strömungsweg umgeschaltet, so daß der Körperhöhlendruck auf dem gewünschten Pegel konstant gehalten wird. Gefährdungen für den Patienten werden auf diese Weise weitgehend vermieden. Weiterhin ist es möglich, Fehlfunktionen des Gerätes und/oder eventuell angeschlossener Zusatzgeräte durch einen Test am Gerät selbst festzustellen, da die von den Druckaufnehmern des Gerätes gemessenen Gasdruckwerte ständig in der Auswerteelektronik verglichen werden und bei Überschreitung ein Alarm ausgelöst wird.

Weitere bevorzugte Merkmale der Erfindung sind aus den Unteransprüchen entnehmbar.

Die Erfindung ist nachstehend anhand eines Ausführungsbeispieles in Form eines in der anliegenden Zeichnung dargestellten Blockschaltbildes näher erläutert.

Nach dem Zeichnungsbeispiel wird $CO_2$ -oder $N_2O$-Gas unter Anzeige des Druckes aus einer Gasflasche 1 über einen Druckminderer 2 mittels des Leitungsteils 3 direkt oder über einen Zwischenbehälter 4 mit Füllstandsmesser 5 geleitet. Bei Entnahme aus dem Behälter 4 wird das Gas über das Ventil 6 einem Druckminderer 7 und dann einem Ventil 8 zugeführt, welches geöffnet wird, so daß das Gas über einen zweiten Druckminderer 9 und eine folgende Drossel 10 oder direkt zum Ventil 12 gelangt.

Zum schnellen Füllen der Körperhöhle eines Patienten wird das Gas dann auf einem ersten Strömungsweg I von einer Flowanzeige 13 über ein Ventil 14, einen Druckminderer 15, ein Umschaltventil 18 und einer Leitung 23 mittels einer die Bauchdecke des Patienten durchdringenden Kanüle 24 in die Bauchhöhle geleitet. Der Druckminderer 15 stellt dabei den Druck auf einen Wert ein, der oberhalb des zu wählenden Druckes in der Körperhöhle liegt, um einerseits einen hohen Gasfluß zu erreichen und um andererseits den Durchflußwiderstand der Schlauchleitung und insbesondere den der Kanüle 24 zu überwinden.

Das schnelle Füllen der Körperhöhle erfolgt jeweils über den ersten Strömungsweg I für eine Zeit T 1 von ca. zwei Sekunden, wobei dieser Druck nicht gemessen wird. Das Umschaltventil 18 wird sodann auf einen zweiten Strömungsweg II umgeschaltet, so daß das Gas nunmehr von der Flowanzeige 13 über einen auf den zu wählenden Körperhöhlendruck, beispielsweise mittels eines Motors 17, einstellbaren Druckminderer 16, einen Flowsensor 25, an dem ein erster Druckaufnehmer 21 mit einer Anzeige 22 angeschlossen ist, und wieder über das Umschaltventil 18 in die Körperhöhle geleitet wird. Das Umschalten des Ventils 18 aufden ersten bzw. zweiten Strömungsweg erfolgt solange, bis der durch den im Strömungsweg II angeordneten Flowsensor 25 während einer Zeitdauer T2 gemessene Flow gleich oder in etwa gleich null ist. Hat der Flow den Wert null oder in etwa diesen Wert erreicht, so bleibt das Umschaltventil 18 ständig auf den Strömungsweg II geschaltet, wodurch die bei geringen Gasverlusten auftretenden Druckschwankungen durch den ständigen Druckausgleich wirksam verhindert werden.

Fließt dagegen über den zweiten Strömungsweg II noch Gas, z.B. mehr als 0,3 l/min, in die Körperhöhle, so bedeutet dies, daß der vorgewählte Druck in der Körperhöhle noch nicht erreicht ist. Dieser Druck wird von einem zweiten Druckaufnehmer 26 in ein elektrisches Signal umgewandelt und von einer Anzeige 27 analog oder digital angezeigt. Es wird dann das Umschaltventil 18 sofort wieder für ca. zwei Sekunden auf den ersten Strömungsweg I umgeschaltet. Fließt nach erneutem Umschalten des Ventils 18 ausdem Druckminderer 18 bzw. durch den Zweiten Strömungsweg II kein Gas mehr zum Patienten, so bedeutet dies, daß der vorgewählte Körperhöhlendruck vollständig oder nahezu vollständig erreicht ist. Das Umschaltventil 18 bleibt dann, wie oben bereits beschrieben, auf den zweiten Strömungsweg umgeschaltet, wobei Gasverluste kleiner als 0,3 l/min ständig ausgeglichen werden. Fällt der Druck infolge größerer Gasverluste, beispielsweise durch einen Instrumentenwechsel oder dergleichen, plötzlich ab, so schaltet das Umschaltventil 18 sofort auf den ersten Strömungsweg I um, um die Körperhöhle schnell so lange zu füllen, bis der vorgewählte Körperhöhlendruck wieder erreicht ist.

Über den Druckaufnehmer 21 des zweiten Strömungsweges II wird der durch den Druckminderer 16 vorgewählte Körperhöhlendruck gemessen und als elektrisches Signal der Anzeige 22 zugeführt, die den Wert des Körperhöhlendruckes digital oder analog anzeigt.

Durch das Vorhandensein der beiden Druckaufnehmer 21, 26 sowie der sich daran anschließenden Anzeigen 22,27 ist es möglich, die ordnungsgemäße Funktion des Insufflationsgerätes zu prüfen, die dann gegeben ist, wenn nach Verschließen der Zuführungsleitung 23 und Inbetriebnahme des Insufflationsgerätes nach einer bestimmten kurzen Zeitdauer gleiche Druckwerte angezeigt werden.

Zur Überprüfung einer möglichen Anzeigeabweichung vom vorgewählten Druck führt das erfindungsgemäße Insufflationsgerät ständig einen Vergleich der beiden durch die Anzeigen 22 und 27 angezeigten Druckwerte durch, wobei eine Auswerteelektronik 28 bei Überschreiten eines vorgewählten Druckes um 658 Pa (5 mmHg) einen ersten Alarm an einem Alarmgerät 31 auslöst.

Wird der vorgewählte Grenzwert um 3948 Pa (30 mmHg) überschritten, so wird ein zweiter Alarm ausgelöst und die Zuführungsleitung 23 und eine Pumpe 29 als auch eventuell weitere, an Ausgängen 32 der Auswerteelektronik 28 angeschlossene und von der Auswerteelektronik 28 angesteuerte Druckausgänge 30 gesperrt bzw. Zusatzgeräte 19,20 außer Betrieb gesetzt. Dieser Zustand wird von einer oder von den beiden Anzeigen 22,27 angezeigt. Eine weitere Zufuhr von Gas in die Körperhöhle kann dann erst nach Beseitigen bzw. Korrektur des vorliegenden Fehlers, der Fehlbedienung oder einer falschen Handhabung erfolgen.

Die Einstellung des Druckminderers 16 auf den gewünschten maximalen Körperhöhlendruck und dessen Messung mittels des Druckaufnehmers 21 und die Messung des Körperhöhlendruckes mittels des Druckaufnehmers 26 verlangen einen ständigen Vergleich der beiden Druckwerte, so daß Störungen sofort erkannt und in der beschriebenen Weise beseitigt werden.

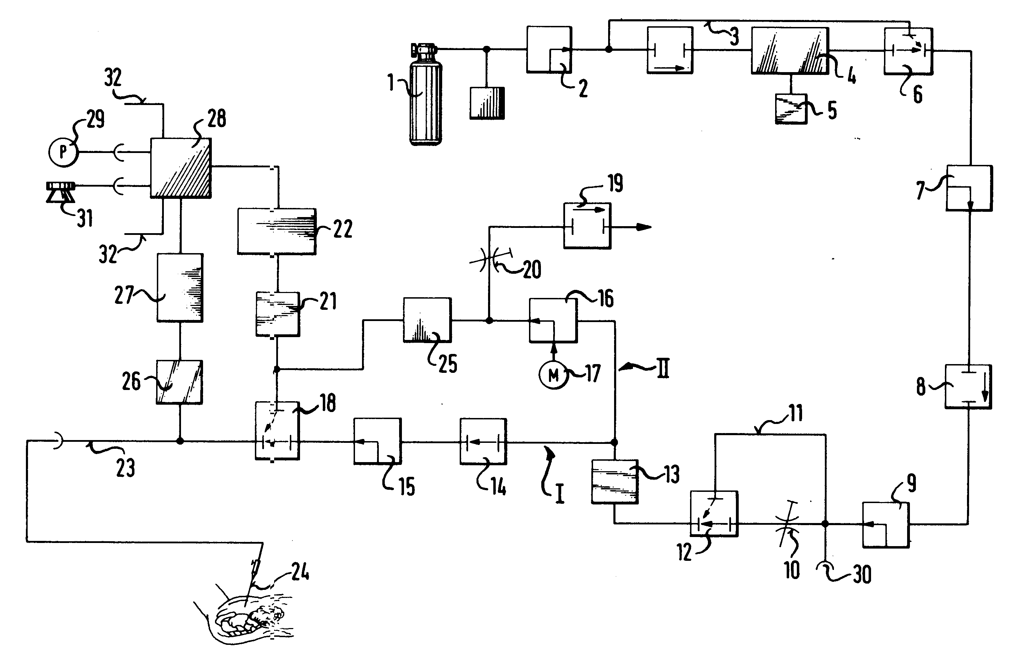

**Patentansprüche**

1. Insufflationsgerät für endoskopische Eingriffe, bei dem ein in eine Körperhöhle eines Lebewesens einzuleitendes Gas aus einer Gasquelle (1) direkt oder über einen Zwischenbehälter (4) mittels einer Zuführungsleitung (23) zugeführt wird, wobei die Leitung (23) wenigstens einen Druckminderer (2,9), wenigstens ein Absperrventil (8), einen Druckaufnehmer (26) und eine Flowanzeige (13) aufweist und wobei die Zuführungsleitung (23) auf einem Streckenabschnitt aus zwei parallelen Strömungswegen (I,II) besteht und über den Druckaufnehmer (26) an eine Auswerteschaltung (28) angeschlossen ist, dadurch gekennzeichnet, daß die beiden Strömungswege (I,II) abströmwärts an die Flowanzeige (13) angeschlossen sind, daß der erste Strömungsweg (I) ein Absperrventil (14), einen das Gas auf einen Wert oberhalb des gewünschten Körperhöhlendruckes einstellenden Druckminderer (15) und ein Umschaltventil (18) aufweist, daß der zweite Strömungsweg (II) einen auf den gewünschten Körperhöhlendruck einstellbaren Druckminderer (16), einen Flowsensor (25) und einen daran angeschlossenen, ersten Druckaufnehmer (21) mit einer Anzeige (22) aufweist und an das Umschaltventil (18) angeschlossen ist, daß abströmwärts des Umschaltventiles (18) ein zweiter Druckaufnehmer (26) mit einer Anzeige (27) an die Zuführungsleitung (23) angeschlossen ist, dessen Wert ständig mit demjenigen des weiteren Druckaufnehmers (21) des zweiten Strömungsweges (II) verglichen wird, und daß zum schnellen Gasfüllen der Körperhöhle der erste Strömungsweg (I) für eine erste Zeitdauer T1 geöffnet und im Anschluß daran das Umschaltventil (18) auf den zweiten Strömungsweg (II) für eine zweite Zeitdauer T2 umgeschaltet wird, innerhalb der die Strömung mittels des Flowsensors (25) überwacht und das Umschaltventil (18) in Abhängigkeit des innerhalb der Zeitdauer T2 gemessenen Gasstromes wahlweise auf den Strömungsweg (I) oder (II) umschaltbar ist.

2. Insufflationsgerät nach Anspruch 1, dadurch gekennzeichnet, daß das Umschaltventil (18) aufden zweiten Strömungsweg (II) umgesteuert wird und umgesteuert bleibt, wenn der innerhalb der Zeitdauer T2 gemessene Flow gleich oder etwa gleich null ist.

3. Insufflationsgerät nach Anspruch 1 und 2, dadurch gekennzeichnet, daß die Auswerteelektronik (28) bei Überschreiten des Körperhöhlendruckes um einen bestimmten vorwählbaren Wert ein erstes Signal auslöst und bei weiterem Überschreiten des Druckes über einen vorwählbaren Grenzwert ein zweites Signal auslöst, die Gaszufuhr in die Körperhöhle und zu einem Druckausgang 30) sperrt und gleichzeitig eine Pumpe (29) und weitere, an den Ausgängen (32) anschließbare und von der Auswerteelektronik (28) ansteuerbare Zusatzgeräte (19) außer Betrieb setzt.

4. Insufflationsgerät nach Anspruch 3, dadurch gekennzeichnet, daß das bei Überschreiten des vorwählbaren Grenzwertes außer Betrieb gesetzte Insufflationsgerät erst nach erfolgter Beseitigung oder Korrektur der die Abschaltung bewirkenden Fehlfunktion, Fehlbedienung oder einer fehlerhaften Handhabung wieder in Betrieb nehmbar ist.

## Claims

1. Insufflation apparatus for endoscopic operations, in which a gas to be introduced into a body cavity of a living being is supplied from a gas source (1) directly or via an intermediate receptacle (4) by means of a feed line (23) having at least one pressure reducer (2,9), at least one shut-off valve (8), a pressure sensor (26) and a flow indicator (13), said feed line (23) consisting, in one section, of two parallel flow paths (I, II) and being connected via the pressure sensor (26) to an evaluation circuit (28), characterized in that the two flow paths (I, II) are connected downstream of the flow indicator (13), in that the first flow path (I) has a shut-off valve (14), a pressure reducer (15) setting the gas to a valve above the desired body cavity pressure and a changeover value (18), in that the second flow path (II) has a pressure reducer (16) which can be set to the desired body cavity pressure, a flow sensor (25) and a first pressure sensor (21), with an indicator (22), connected to said flow sensor (25) and is connected to the changeover valve (18), in that a second pressure sensor (26) with indicator (27) is connected to the feed line (23) downstream of the changeover valve (18), its value being continuously compared with that of the other pressure sensor (21) of the second flow path (II), and in that, for rapid filling of the body cavity with gas, the first flow path (I) is opened for a first time period T1 after which the changeover valve (18) is switched to the second flow path (II) for a second time period T2 during which the flow is monitored by means of the flow sensor (25) and the changeover valve (18) can be switched to flow path (I) or (II) as desired in dependence on the gas flow measured during time period T2.

2. Insufflation apparatus according to Claim 1, characterized in that the changeover valve (18) is switched to the second flow path (II) and remains switched to it when the flow measured during time period T2 is equal to, or approximately equal to, zero.

3. Insufflation apparatus according to Claims 1 and 2, characterized in that if the body cavity pressure is exceeded by a set amount, which can be preselected, the electronic evaluation circuit (28) releases a first signal, and if the pressure is further exceeded beyond a limit value, which can be preselected, it releases a second signal, shuts off the gas supply to the body cavity and to a pressure outlet (30) and simultaneously switches off a pump (29) and further ancillary equipment (19) which may be connected to the outlets (32) and can be controlled by the electronic evaluation circuit (28).

4. Insufflation apparatus according to Claim 3, characterised in that once the insufflation apparatus has been put out of operation owing to the preselected limit value being exceeded it cannot be put back into operation until the malfunction or incorrect operation or handling which caused the disconnection has been eliminated or rectified.

**Revendications**

1. Dispositif d'insufflation pour interventions endoscopiques dans lequel un gaz à introduire dans une cavité corporelle d'un être vivant est amené à partir d'une source de gaz (1) au moyen d'une conduite d'amenée (23), directement ou en passant par un réservoir intermédiaire (4), la conduite (23) comportant au moins un détendeur (2, 9), au moins une vanne d'arrêt (8), un capteur de pression (26) et une signalisation de débit (13) et la conduite d'amenée (23) se composant, sur une partie de son parcours, de deux trajets d'écoulement parallèles (I, II) et se raccordant par l'intermédiaire du capteur de pression (26) à un dispositif d'évaluation (28), caractérisé en ce que les deux trajets d'écoulement (I, II) sont raccordés en aval à la signalisation de débit (13), en ce que le premier trajet d'écoulement (I) comporte une vanne d'arrêt (14) un détendeur (15) réglant le gaz à une pression supérieure à la pression souhaitée dans la cavité corporelle et une vanne de commutation (18), en ce que le deuxième trajet d'écoulement (II) comporte un détendeur (16) réglable à la pression souhaitée dans la cavité corporelle, un détecteur de débit (25) et un premier capteur de pression (21) à signalisation (22) et est raccordé à la valve de commutation (18), en ce qu'il est raccordé à la conduite d'amenée (23), en aval de la vanne de commutation (18), un deuxième capteur de pression (26) à signalisation (27), dont la valeur est constamment comparée avec celle de l'autre capteur de pression (21) du deuxième trajet d'écoulement (II), et en ce que pour le remplissage de gaz rapide de la cavité corporelle le premier trajet d'écoulement (I) est ouvert pendant un premier laps de temps T1 et la vanne de commutation (18) est consécutivement commutée sur le deuxième trajet d'écoulement (II) pendant un deuxième laps de temps T2 au cours duquel l'écoulement est surveillé au moyen du détecteur d'écoulement (25) et la vanne de commutation (18) peut être commutée au choix sur le trajet d'écoulement (I) ou (II) en fonction du flux gazeux mesuré à l'intérieur du laps de temps T2.

2. Dispositif d'insufflation selon la revendication 1, caractérisé en ce que la vanne de commutation (18) est inversée sur le deuxième trajet d'écoulement (II) et reste inversée lorsque le flux mesuré au cours du laps de temps T2 est égal ou à peu près égal à zéro.

3. Dispositif d'insufflation selon la revendication 1 ou 2, caractérisé en ce que l'électronique d'évaluation (28) déclenche un premier signal lorsque la pression de la cavité corporelle est dépassée d'une valeur définie qui peut être choisie au préalable et déclenche un deuxième signal lorsque lors d'un dépassement plus poussé de la pression au-delà d'une valeur limite qui peut être choisie au préalable, arrête l'amenée de gaz dans la cavité corporelle et à une sortie de pression (30) et met simultanément hors fonction une pompe (29) et d'autres dispositifs additionnels (19) qui peuvent être raccordés aux sorties (32) et être commandés par l'électronique d'évaluation (28).

4. Dispositif d'insufflation selon la revendication 3, caractérisé en ce que le dispositif d'insufflation qui a été mis hors fonction lors du dépassement de la valeur limite qui peut être choisie au préalable ne peut être remis en fonction qu'après élimination ou correction exécutée du fonctionnement défectueux, de la commande défectueuse ou d'une manipulation défectueuse qui a provoqué la mise hors fonction.